# FASCICULE DE BREVET EUROPEEN

(11) **EP 0 932 602 B1**
(45) Date de publication et mention de la délivrance du brevet: **03.03.2004**
(21) Numéro de dépôt: 98938746.9
(22) Date de dépôt: 13.07.1998
(51) Int. Cl.: C07D 233/00

(54) **BASES D'OXYDATION CATIONIQUES, LEUR UTILISATION POUR LA TEINTURE D'OXYDATION DES FIBRES KERATINIQUES, COMPOSITIONS TINCTORIALES ET PROCEDES**
BASEN ZUR KATIONISCHEN OXIDATION, DEREN ANWENDUNG ZUR OXIDATIVEN FÄRBUNG KERATINISCHER FASERN, FÄRBEMITTEL UND FÄRBUNGSVERFAHREN
BASES OF CATIONIC OXIDATION, THEIR USE FOR OXIDATIVE DYEING OF KERATIN FIBRES, DYE COMPOSITIONS AND DYEING PROCESSES

(30) Priorité: 16.07.1997 FR 9709029
(43) Date de publication de la demande: 04.08.1999
(73) Titulaire: L'OREAL, 75008 Paris (FR)
(72) Inventeur: GENET, Alain, F-93600 Aulnay-sous-Bois (FR); LAGRANGE, Alain, F-77770 Coupvray (FR)
(74) Mandataire: Goulard, Sophie
(86) Numéro de dépôt international: PCT/FR1998/001536
(87) Numéro de publication internationale: WO 1999/003834

(56) Documents cités:
- EP-A- 0 360 644
- EP-A- 0 544 400
- EP-A- 0 634 164
- EP-A- 0 673 641
- EP-A- 0 728 463
- WO-A-95/12585
- WO-A-95/15144
- WO-A-97/39727
- FR-A- 2 213 968
- FR-A- 2 217 390
- GB-A- 1 211 801
- US-A- 4 975 092
- US-A- 5 135 543

## Description

L'invention a pour objet de nouvelles bases d'oxydation di-benzéniques comportant au moins un groupement cationique Z, Z étant choisi parmi des chaînes aliphatiques quaternisées, des chaînes aliphatiques comportant au moins un cycle saturé quaternisé, et des chaînes aliphatiques comportant au moins un cycle insaturé quaternisé, leur utilisation pour la teinture d'oxydation des fibres kératiniques, les compositions tinctoriales les contenant, ainsi que les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs. La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements). Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans le brevet US 5,139,532, d'utiliser certains dérivés cationiques de paraphénylènediamines, à savoir plus précisément des paraphénylènediamines dont un des groupements amino est monosubstitué par une chaîne aliphatique quaternisée, pour la teinture d'oxydation des fibres kératiniques dans des nuances intenses et plus rouges que celles obtenues habituellement en mettant en oeuvre des paraphénylènediamines classiques, c'est à dire ne portant pas de groupement cationique. Toutefois, l'utilisation des paraphénylènediamines décrites dans ce brevet antérieur ne permet pas d'obtenir une riche palette de couleurs et, de plus, les colorations obtenues ne donnent pas toujours entière satisfaction du point de vue de leur résistance vis à vis des diverses agressions que peuvent subir les cheveux (action de la lumière, de la transpiration, des shampooings, etc...). On connaît des bases d'oxydation du type bis-phénylènediamines non cationiques dans la teinture capillaire dans les demandes EP 728 463, EP 360 644 et EP 634 164.

Or, la demanderesse vient maintenant de découvrir, de façon totalement inattendue et surprenante, que certaines nouvelles bases d'oxydation di-benzéniques de formule (I) ci-après définie, comportant au moins un groupement cationique Z, Z étant choisi parmi des chaînes aliphatiques quaternisées, des chaînes aliphatiques comportant au moins un cycle saturé quaternisé, et des chaînes aliphatiques comportant au moins un cycle insaturé quaternisé, non seulement conviennent pour une utilisation comme précurseurs de colorant d'oxydation, mais en outre qu'elles permettent d'obtenir des compositions tinctoriales conduisant à des colorations puissantes, dans une large palette de couleurs, et présentant d'excellentes propriétés de résistances aux différents traitements que peuvent subir les fibres kératiniques. Enfin, ces composés s'avèrent être aisément synthétisables.

Ces découvertes sont à la base de la présente invention.

L'invention a donc pour premier objet de nouveaux composés de formule (I) suivante, et leurs sels d'addition avec un acide : 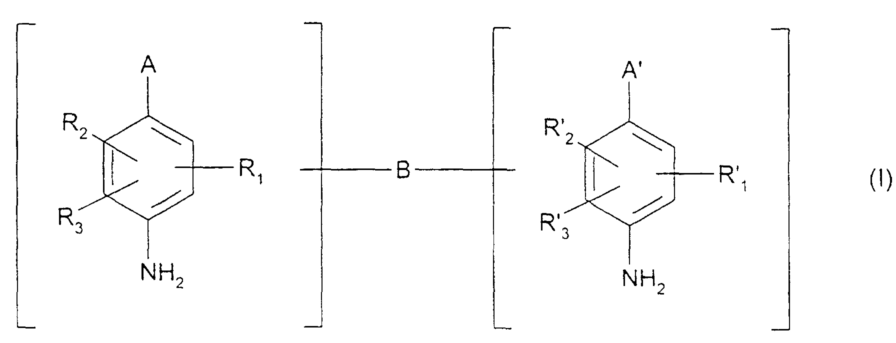 dans laquelle :
- R₁, R₂, R₃, R'₁, R'₂ et R'₃, qui peuvent être identiques ou différents, représentent ; un atome d'halogène ; un radical alkyl(C₁-C₆) carbonyle ; un radical aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; ou un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, alkyl(C₁-C₆) carboxy, carbamyle, N-alkyl(C₁-C₆)carbamyle, N,N-dialkyl(C₁-C₆)carbamyle, alkyl(C₁-C₆)sulfonyle, aminosulfonyle, N-alkyl(C₁-C₆)aminosulfonyle, N,N-dialkyl(C₁-C₆)aminosulfonyle, thiocarbamyle, formyle; R₁ et R_{1'} peuvent former ensemble un bras de liaison B ;
- R₆ désigne un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle ;
- A représente un groupement -NR₄R₅ ou un radical hydroxyle ;
- A' représente un groupement -NR'₄R'₅ ou un radical hydroxyle ;
- R₄, R₅, R'₄ et R'₅, identiques ou différents, représentent un atome d'hydrogène ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle; R⁴ et R_{4'} peuvent former ensemble un bras de liaison B ;
- Z est choisi parmi les cycles pyrrolique, imidazolique, pyrazolique, oxazolique, thiazolique et triazolique, pyridinium, pyrimidinium, pyrazinium, oxazinium, triazinium, pyrrolidinium, pipéridinium, pipérazinum ou morpholinium
- B est un bras de liaison qui relie AI à AII et qui représente une chaîne alkylène comportant de préférence de 1 à 14 atomes de carbone, linéaire ou ramifiée pouvant être interrompue par un ou plusieurs groupements Z et/ou par un ou plusieurs hétéroatomes choisis parmi les atomes d'oxygène, de soufre ou d'azote, et éventuellement substituée par un ou plusieurs radicaux hydroxyle ou alcoxy en C₁-C₆, et pouvant porter une ou plusieurs fonctions cétone ;
étant entendu :
- que le nombre de groupement cationique Z est au moins égal à 1 ;
- qu'il existe un seul bras de liaison entre AI et AII.

Comme indiqué précédemment, les colorations obtenues avec la composition de teinture d'oxydation conforme à l'invention sont puissantes et permettent d'atteindre une large palette de couleurs. Elles présentent de plus d'excellentes propriétés de résistance vis à vis de l'action des différents agents extérieurs (lumière, intempéries, lavage, ondulation permanente, transpiration, frottements). Ces propriétés sont particulièrement remarquables notamment en ce qui concerne la résistance des colorations obtenues vis à vis de l'action de la lumière, des lavages, de l'ondulation permanente et de la transpiration.

Dans la formule (I) ci-dessus les radicaux alkyle et alcoxy peuvent être linéaires ou ramifiés.

Parmi les composés de formule (I) ci-dessus, on peut notamment citer :
- le dichlorure de 1,3-bis-{3{3'[(4"-amino-3"-méthyl-aniline)-N-propyl]}-3H-imidazol-1-ium}-propane ;
- le dichlorure de 1,3-bis-{3{3'[(4"-amino-2"-méthyl-aniline)-N-propyl]}-3H-imidazol-1-ium}-propane, monohydrate, diéthanol ;
- le dichlorure de 1,3-bis-{3{3'[(4"-amino-aniline)-N-propyl]}-3H-imidazol-1-ium}-propane, monohydrate, éthanol ;
- le dichlorure de 1,3-bis-{3{3'[(4"-amino-aniline)-N-propyl]}-3H-imidazol-1-ium}-2-propanol, monohydrate ;
- le dichlorure de 1,4-bis-{3[3-(2,5-diamino-phénoxy)-propyl]-3H-imidazol-1-ium}- butane, dihydrate ;
- le monochlorure de 1,3-bis-[3-(2,5-diamino-phénoxy)-propyl]-3H-imidazol-1-ium, monohydrate ;
- le dichlorure de 1,4-bis-[3-(5-amino-2-hydroxy-benzyl)-3H-imidazol-1ium]-butane, monohydrate ;
- le dichlorure de 1,3-bis-{3-[3-(2,5-diamino-phénoxy)-propyl]-3H-imidazol-1-ium}-propane, dihydrate ;
- le dichlorure de 1,3-bis-{3{3'[(4"-amino-aniline)-N-propyl]}-3H-imidazol-1-ium}-propane ;
- le dichlorure de 1,3-bis-{4{4'(4-[3-(4"-amino-phénylamino)-propyl]}-1,3-diméthyl-3H-imidazol-1-ium}-propane,
- le dichlorure de 1,3-bis-{4{4'(4-[3-(4"-amino-2"-méthyl-aniline)-propyl]}-1,3-diméthyl-3H-imidazol-1-ium}-propane ;
- le monochlorure de 7[2-(2,5-diamino-phénoxy)-éthyl]-3-[3-(2,5-diaminophénoxy)-propyl]-1-méthyl-3-imidazol-1-ium ;
- le monochlorure de 4-[2-(2,5-diamino-phénoxy)-éthyl]-1-[3-(2,5-diaminophénoxy)-propyl]-3-méthyl-3-imidazol-1-ium ;
et leurs sels d'addition avec un acide.

Parmi ces composés de formule (I), on préfère plus particulièrement :
- le dichlorure de 1,3-bis-{3-{3'-[(4"-amino-aniline)-N-propyl]}-3H-imidazol-1-ium}-propane, monohydrate, éthanol ;
- le monochlorure de 1,3-bis-[3-(2,5-diamino-phénoxy)-propyl]-3H-imidazol-1-ium, monohydrate;
- le dichlorure de 1,4-bis-[3-(5-amino-2-hydroxy-benzyl)-3H-imidazol-1-ium]-butane, monohydrate ;
- le dichlorure de 1,3-bis-{3{3'[(4"-amino-aniline)-N-propyl]}-3H-imidazol-1-ium}-propane ;
et leurs sels d'addition avec un acide.

Les composés de formule (I) conformes à l'invention peuvent être facilement obtenus, selon des méthodes bien connues de l'état dé la technique :
- soit par réduction des composés nitrés di-benzéniques cationiques correspondants (para-nitranilines cationiques et/ou para-nitrophénols cationiques),
- soit par réduction des composés nitrosés cationiques correspondants (obtenus par exemple par nitrosation d'une aniline tertiaire ou d'un phénol correspondants),
- soit par réduction des composés azoïques cationiques correspondants (coupure réductrice).

Cette étape de réduction (obtention d'une amine aromatique primaire) qui confère au composé synthétisé son caractère de composé oxydable (de base d'oxydation) suivie ou non d'une salification, est en général, par commodité, la dernière étape de la synthèse.

Cette réduction peut intervenir plus tôt dans la suite des réactions conduisant à la préparation des composés de formule (I), et selon des procédés bien connus il faut alors "protéger" l'amine primaire créée (par exemple par une étape d'acétylation, de benzènesulfonation, etc...), faire ensuite la ou les substitutions ou modifications désirées (y compris la quaternisation) et terminer par le "déprotection" (en général en milieu acide) de la fonction amine.

De même la fonction phénolique peut être protégée selon des procédés bien connus par un radical benzyle ("déprotection" par réduction catalytique) ou par un radical acétyle ou mésyle ("déprotection" en milieu acide).

Lorsque la synthèse est terminée, les composés de formule (I) conformes à l'invention peuvent, le cas échéant, être récupérés par des méthodes bien connues de l'état de la technique telles que la cristallisation, la distillation.

Un autre objet de l'invention est l'utilisation des composés de formules (I) conformes à l'invention à titre de base d'oxydation pour la teinture d'oxydation des fibres kératiniques, et en particulier des fibres humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend à titre de base d'oxydation, dans un milieu approprié pour la teinture, au moins un composé de formule (I) conforme à l'invention.

Le ou les composés de formule (I) conformes à l'invention représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 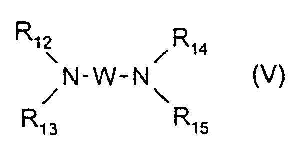 dans laquelle W est un reste propylène éventuellement substitué par un groupement hydroxyle ou un radical alkyle en C₁-C₆ ; R¹², R¹³, R¹⁴ et R¹⁵, identiques ou différents, représentent un atome d'hydrogène, un radical alkyle en C₁-C₆ ou hydroxyalkyle en C₁-C₆.

La composition tinctoriale conforme à l'invention peut encore contenir, en plus des colorants définis ci-dessus, au moins une base d'oxydation additionnelle qui peut être choisie parmi les bases d'oxydation classiquement utilisées en teinture d'oxydation et parmi lesquelles on peut notamment citer les paraphénylènediamines, les bis-phénylalkylènediamines différentes des composés de formule (I) conformes à l'invention, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, les paraphénylènediamines décrites dans la demande de brevet français FR 2 630 438, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols, on peut plus particulièrement citer à titre d'exemple, citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation additionnelles et coupleurs) sont notamment choisis parmi les chlorhydrates, les bromhydrates, les sulfates, les citrates, les succinates, les tartrates, les lactates et les acétates.

La composition tinctoriale conforme l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

### EXEMPLES DE PREPARATION

### EXEMPLE DE PREPARATION 1 : Synthèse du dichlorure 1,3-bis-{3{3'[(4"-amino-3"-méthyl-aniline)-N-propyl]}-3H-imidazol-1-ium}-propane, tétrachlorhydrate, 1/3 d'éthanol, monohydrate

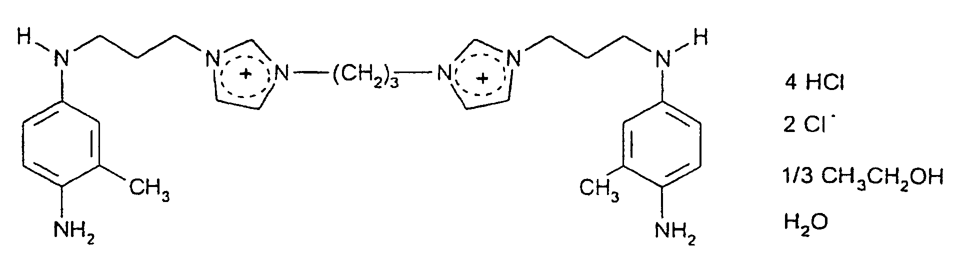

### a) Préparation de la (3-imidazol-1-yl-propyl)-(3-méthyl-4-nitro-phényl)-amine

On a chauffé à 90°C un mélange de 125,5 g (1 mole) de 3-imidazol-1-yl-propylamine et de 41,4 g (0,3 mole) de carbonate de potassium dans 140 ml d'eau.

On a ajouté goutte à goutte 77,6 g (0,5 mole) de 4-fluoro-2-méthyl-1-nitro-benzène en 45 minutes et maintenu à une température de 90-95°C pendant 2 heures. On a refroidi le mélange réactionnel dans un bain de glace, essoré le précipité cristallisé, lavé à l'eau et séché à 40°C sous vide sur anhydride phosphorique.

Après recristallisation de l'éthanol absolu au reflux on a obtenu 96 g de cristaux jaunes qui ont fondu à 133°C (Kofler) et dont l'analyse élémentaire calculée pour C₁₃H₁₆N₄O₂ était :

| % | C | H | N | O |
|---|---|---|---|---|
| Calculé | 59,99 | 6,20 | 21,52 | 12,29 |
| Trouvé | 59,55 | 6,22 | 21,43 | 12,88 |

### b) Quaternisation de la (3-imidazol-1-yl-propyl)-(3-méthyl-4-nitro-phényl)-amine

On a chauffé pendant 6 heures au reflux un mélange de 88,9 g (0,341 mole) de (3-imidazol-1-yl-propyl)-(3-méthyl-4-nitro-phényl)-amine obtenue à l'étape précédente et de 19,3 g (0,1705 mole) de 1,3-dichloro-propane dans 220 ml de pentanol normal.

Le milieu réactionnel était une solution que l'on a refroidi dans un bain de glace.

Une gomme a précipité puis recristallisé en masse. On a essoré, lavé avec de l'éthanol absolu, recristallisé de l'éthanol à 95° au reflux et séché à 40°C sous vide. On a obtenu 56 g de cristaux jaunes du produit attendu qui ont fondu à 138-140°C (Kofler) et dont l'analyse élémentaire calculée pour C₂₉H₃₈N₈O₄Cl₂ + H₂O était :

| % | C | H | N | O | Cl |
|---|---|---|---|---|---|
| Calculé | 53,46 | 6,19 | 17,20 . | 12,28 | 10,88 |
| Trouvé | 52,69 | 6,25 | 17,06 | 12,89 | 10,99 |

### c) Préparation du dichlorure 1,3-bis-{3{3'[(4"-amino-3"-méthyl-aniline)-N-propyl]}-3H-imidazol-1-ium}-propane, tétrachlorhydrate

Dans un hydrogénateur, on a placé 43 g (0,068 mole) du produit obtenu ci-dessus à l'étape précédente, 2 g de palladium à 5% sur charbon (contenant 50% d'eau) et 170 ml d'eau.

La réduction s'est faite en une heure sous une pression d'hydrogène d'environ 5 bars et à une température qui a été portée progressivement à 75°C.

Après filtration du catalyseur sous azote on a coulé sur de l'acide chlorhydrique aqueux.

On a évaporé le filtrat à sec sous pression réduite et repris le composé partiellement cristallisé dans l'éthanol absolu jusqu'à cristallisation complète.

Après séchage à 40°C sous vide et sur potasse on a obtenu 38,4 g de cristaux blancs qui ont fondu avec décomposition à 146-160°C (Kofler) et dont l'analyse élémentaire calculée pour C₂₉H₄₆N₈Cl₆ + H₂O + 1/3 CH₃CH₂OH était :

| % | C | H | N | O | Cl |
|---|---|---|---|---|---|
| Calculé | 47,33 | 6,69 | 14,88 | 2,83 | 28,26 |
| Trouvé | 48,10 | 6,74 | 14,69 | 2,86 | 27,99 |

### EXEMPLE DE PREPARATION 2 : Synthèse du dichlorure de 1,3-bis-{3{3'[(4"-amino-2"-méthyl-aniline)-N-propyl]}-3H-imidazol-1-ium}-propane, tétrachlorhydrate, monohydrate, diéthanol

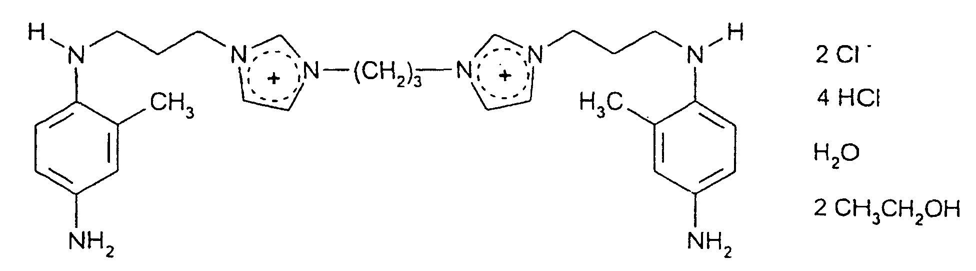

### a) Préparation de la (3-imidazol-1-yl-propyl)-(2-méthyl-4-nitro-phényl)-amine

On a chauffé à 90°C un mélange de 250,4 g (2 moles) de 3-imidazol-1-yl-propylamine et de 82,8 g (0,6 mole) de carbonate de potassium dans 280 ml d'eau. On a ajouté goutte à goutte 155,1 g (1 mole) de 1-fluoro-2-méthyl-4-nitro-benzène en 30 minutes et maintenu à une température de 90-95°C pendant 4 heures.

On a refroidi le mélange réactionnel dans un bain de glace, essnré le précipité cristallisé, lavé à l'isopropanol et séché à 40°C sous vide.

Après recristallisation de l'éthanol à 96° au reflux on a obtenu 144,8 g de cristaux orangés qui ont fondu à 163°C (Kofler) et dont l'analyse élémentaire calculée pour C₁₃H₁₆N₄O₂ était :

| % | C | H | N | O |
|---|---|---|---|---|
| Calculé | 59,99 | 6,20 | 21,52 | 12,29 |
| Trouvé | 59,60 | 6,15 | 21,46 | 13,12 |

### b) Quaternisation de la (3-imidazol-1-yl-propyl)-(2-méthyl-4-nitro-phényl)-amine

On a chauffé pendant 6 heures au reflux un mélange de 130,1 g (0,5 mole) de (3-imidazol-1-yl-propyl)-(2-méthyl-4-nitro-phényl)-amine obtenue ci-dessus à l'étape précédente et de 28,25 g (0,25 mole) de 1,3-dichloro-propane dans 320 ml de pentanol normal.

Le milieu réactionnel était une solution que l'on a refroidi dans un bain de glace et à laquelle on a ajouté de l'éthanol absolu : une gomme a précipité puis cristallisé.

On a essoré, lavé avec de l'éthanol absolu et séché à 40°C sous vide.

On a obtenu 129,6 g de cristaux jaunes qui ont fondu à 148-150°C (Kofler) et dont l'analyse élémentaire calculée pour C₂₉H₃₈N₈O₄Cl₂ + 2,5H₂O était :

| % | C | H | N | O | Cl |
|---|---|---|---|---|---|
| Calculé | 51,33 | 6,39 | 16,51 | 15,32 | 10,45 |
| Trouvé | 51,69 | 6,45 | 16,62 | 15,38 | 10,33 |

### c) Préparation du dichlorure de 1,3-bis-{3{3'[(4"-amino-2"-méthyl-aniline)-N-propyl]}-3H-imidazol-1-ium}-propane, tétrachlorhydrate, monohydrate, diéthanol

On a utilisé le mode opératoire décrit ci-dessus à l'exemple 1, étape c).

A partir de 100 g (0,1578 mole) du produit obtenu ci-dessus à l'étape précédente, on a obtenu 111,0 g de cristaux blancs qui ont fondu avec décomposition à 165-170°C (Kofler) et dont l'analyse élémentaire calculée pour C₂₉H₄₆N₈Cl₆ + H₂O + 2CH₃CH₂OH était :

| % | C | H | N | O | Cl |
|---|---|---|---|---|---|
| Calculé | 47,78 | 7,29 | 13,51 | 5,79 | 25,64 |
| Trouvé | 47,89 | 7,23 | 13,94 | 5,82 | 26,43 |

### EXEMPLE DE PREPARATION 3 : Synthèse du dichlorure de 1,3-bis-{3-{3'-[(4"-amino-aniline)-N-propyl]}-3H-imidazol-1-ium}-propane, tétrachlorhydrate, monohydrate, éthanol

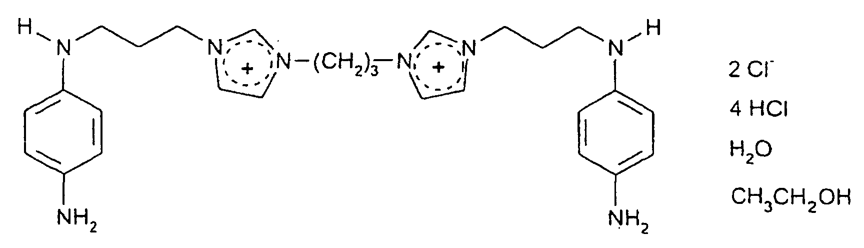

### a) Préparation de la (3-imidazol-1-yl-propyl)-(4-nitro-phényl)-amine

On a chauffé au bain-marie bouillant un mélange de 150,2 g (1,2 moles) de 3-imidazol-1-yl-propylamine et de 139,4 ml (1 mole) de triéthylamine dans 200 ml de dioxane.

On a ajouté goutte à goutte 141,1 g (1 mole) de 1-fluoro-4-nitro-benzène en 30 minutes et maintenu à une température de 90-95°C pendant 1 heure.

On a versé dans 2 kg d'eau glacée, essoré le précipité cristallisé, lavé à l'eau et recristallisé de l'éthanol à 96° au reflux.

On a obtenu 106,0 g de cristaux jaunes qui ont fondu à 126°C (Kofler) et dont l'analyse élémentaire calculée pour C₁₂H₁₄N₄O₂ était :

| % | C | H | N | O |
|---|---|---|---|---|
| Calculé | 58,53 | 5,73 | 22,75 | 12,99 |
| Trouvé | 58,33 | 5,83 | 22,81 | 13,41 |

### b) Préparation du dichlorure de 1,3-bis-{3-{3'-[(4"-nitro-aniline)-N-propyl]}-3H-imidazol-1-ium}-propane, 1/3 hydrate

On a chauffé pendant 6 heures au reflux un mélange de 39,4 g (0,16 mole) de (3-imidazol-1-yl-propyl)-(4-nitro-phényl)-amine obtenue à l'étape précédente et de 9,03 g (0,08 mole) de 1,3-dichloro-propane dans 160 ml de toluène. Une gomme en suspension a cristallisé.

On a refroidi, essoré le précipité cristallisé, réempaté deux fois dans le minimum d'éthanol absolu et séché à 45°C sous vide.

On a obtenu 23,3 g de cristaux jaunes qui ont fondu à 186°C (Kofler) et dont l'analyse élémentaire calculée pour C₂₇H₃₄N₈O₄Cl₂ + 1/3 H₂O était :

| % | C | H | N | O | Cl |
|---|---|---|---|---|---|
| Calculé | 53,03 | 5,71 | 18,32 | 11,34 | 11,59 |
| Trouvé | 53,00 | 5,68 | 18,33 | 11,19 | 11,44 |

### c) Réduction du dichlorure de 1,3-bis-{3-{3'-[(4"-nitro-aniline)-N-propyl]}-3H-imidazol-1-ium}-propane, 1/3 hydrate

On a utilisé le mode opératoire décrit ci-dessus pour l'exemple 1, étape c).

A partir de 46,7 g (0,0771 mole) de dichlorure de 1,3-bis-{3-{3'-[(4"-nitro-aniline)-N-propyl]}-3H-imidazol-1-ium}-propane, 1/3 hydrate, on a obtenu 28,9 g de cristaux blancs qui ont fondu vers 148°C puis vers 180°C (Kofler) et dont l'analyse élémentaire calculée pour C₂₇H₄₂N₈Cl₆ + H₂O + CH₃CH₂OH était :

| % | C | H | N | O | Cl |
|---|---|---|---|---|---|
| Calculé | 46,11 | 6,67 | 14,83 | 4,24 | 28,16 |
| Trouvé | 46,33 | 6,67 | 14,83 | 4,52 | 28,50 |

### EXEMPLE DE PREPARATION 4 : Synthèse du dichlorure de 1,3-bis-{3-{3'-[(4"-amino-aniline)-N-propyl]}-3H-imidazol-1-ium}-2-propanol, tétrachlorhydrate, monohydrate

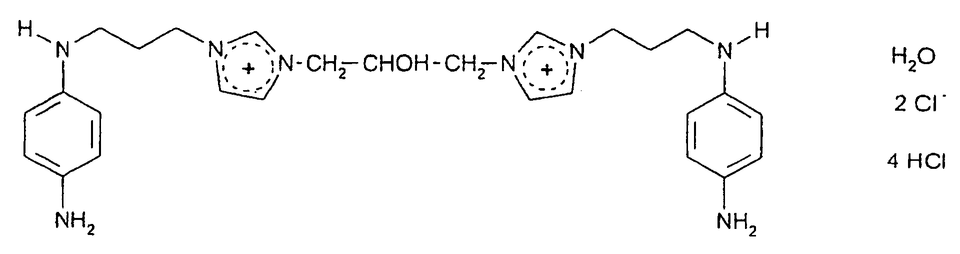

### a) Préparation du dichlorure de 1,3-bis-4-{3-{3'-[(4"-nitro-aniline)-N-propyl]}-3H-imidazol-1-ium}-2-propanol

On a chauffé pendant 8 heures au reflux un mélange de 24,6 g (0,1 mole) de (3-imidazol-1-yl-propyl)-(4-nitro-phényl)-amine obtenue ci-dessus à l'étape a) de l'exemple 3 et de 6,45 g (0,05 mole) de 1,3-dichloro-propan-2-ol dans 100 ml de toluène. Une gomme en suspension a cristallisé.

On a refroidi, essoré le précipité cristallisé, réempaté deux fois dans le minimum d'éthanol absolu et séché à 45°C sous vide.

On a obtenu 24,8 g de cristaux jaunes qui ont fondu à 228-230°C (Kofler) et dont l'analyse élémentaire calculée pour C₂₇H₃₄N₈O₅Cl₂ était :

| % | C | H | N | O | Cl |
|---|---|---|---|---|---|
| Calculé | 52,18 | 5,51 | 18,03 | 12,87 | 11,41 |
| Trouvé | 52,23 | 5,55 | 18,03 | 12,80 | 11,44 |

### b) Réduction du dichlorure de 1,3-bis-{3-{3'-[(4"-nitro-aniline)-N-propyl]}-3H-imidazol-1-ium}-2-propanol

On a utilisé le mode opératoire décrit ci-dessus pour l'exemple 1, étape c).

A partir de 14,8 g (0,0238 mole) de dichlorure de 1,3-bis-{3-{3'-[(4"-nitro-aniline)-N-propyl]}-3H-imidazol-1-ium}-2-propanol, obtenu ci-dessus à l'étape précédente, on a obtenu 8,9 g de cristaux blanc crème qui ont fondu avec décomposition à 160-170°C (Kofler) et dont l'analyse élémentaire calculée pour C₂₇H₄₂N₈OCl₆ + H₂O était :

| % | C | H | N | O | Cl |
|---|---|---|---|---|---|
| Calculé | 44,71 | 6,11 | 15,45 | 4,41 | 29,32 |
| Trouvé | 44,90 | 6,18 | 15,43 | 4,71 | 29,61 |

### EXEMPLE DE PREPARATION 5 : Synthèse du dichlorure de 1,4-bis-{3-[3-(2,5-diamino-phénoxy)-propyl]-3H-imidazol-1-ium}-butane, tétrachlorhydrate, dihydrate

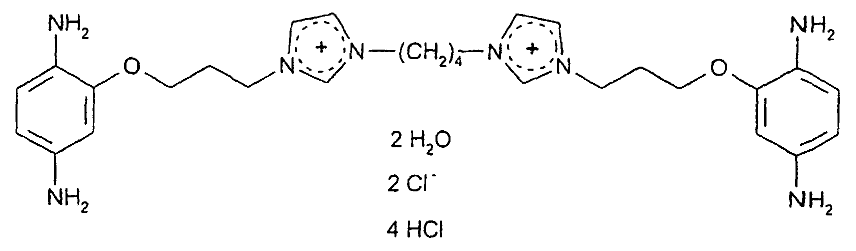

### a) Préparation du N-[2-(3-chloro-propoxy)-4-nitro-phényl]-acétamide

Sous agitation, on a chauffé à 50°C un mélange de 98,1 g (0,5 mole) de N-(2-hydroxy-4-nitro-phényl)-acétamide et de 69,2 g (0,5 mole) de carbonate de potassium dans 500 ml de diméthylformamide, puis on a ajouté 113,0 g ( 1 mole) de 1,3-dichloro-propane et on a continué a chauffer à 50°C pendant 1 heure.

On a versé le mélange réactionnel dans 4 litres d'eau glacée, essoré le précipité cristallisé, réempaté dans l'eau puis dans l'alcool isopropylique et séché sous vide à 40°C sur anhydride phosphorique.

On a obtenu 113,5 g de cristaux beiges qui, après purification par recristallisation de l'acétate d'isopropyle au reflux, ont fondu à 121°C et dont l'analyse élémentaire était conforme à celle calculée pour C₁₁H₁₃N₂O₄Cl.

### b) Quaternisation du N-[2-(3-chloro-propoxy)-4-nitro-phényl]-acétamide

On a chauffé au reflux pendant 11 heures 54,5 g (0,2 mole) de N-[2-(3-chloro-propoxy)-4-nitro-phényl]-acétamide obtenu ci-dessus à l'étape précédente et 19,0 g (0,1 mole) de 1,4-di-imidazol-1-yl-butane dans 160 ml de 2-méthyl-1-propanol.

On a refroidi à température ambiante, décanté le précipité huileux et repris dans de l'éthanol absolu jusqu'à cristallisation.

Après essorage, recristallisation dans l'éthanol absolu au reflux et séchage à 40°C sur potasse on a obtenu 65,9 g de cristaux jaune pâle qui ont fondu à 132-134°C (Kofler) et dont la RMN 1H était conforme.

### c) Préparation du dichlorure de 1,4-bis-{3-[3-(2,5-diamino-phénoxy)-propyl]-3H-imidazol-1-ium}-butane, tétrachlorhydrate, dihydrate

On a utilise le mode opératoire décrit pour l'exemple 1, étape c), sans salifier le composé réduit.

A partir de 77,7 g (0,105 mole) du composé obtenu ci-dessus à l'étape précédente, on a obtenu 59,0 g d'une huile brune.

Ce composé huileux a été mis en solution dans 110 ml d'acide chlorhydrique aqueux à 36% et chauffé pendant 45 minutes au bain-marie bouillant.

On a refroidi à température ambiante (solution), dilué avec 100 ml d'éthanol et refroidi de nouveau dans un bain de glace.

Le précipité cristallisé a été essoré, lavé à l'éthanol absolu et séché sous vide sur potasse.

On a obtenu 34,0 g de cristaux blancs qui ont fondu avec décomposition à 230°C (Kofler) et dont l'analyse élémentaire calculée pour C₂₈H₄₄N₈O₂Cl₆ + 2H₂O était :

| % | C | H | N | O | Cl |
|---|---|---|---|---|---|
| Calculé | 43,48 | 6,26 | 14,49 | 8,27 | 27,50 |
| Trouvé | 43,71 | 6,18 | 14,48 | 8,06 | 27,80 |

### EXEMPLE DE PREPARATION 6 : Synthèse du monochlorure de 1,3-bis-[3-(2,5-diamino-phénoxy)-propyl]-3H-imidazol-1-ium, tétrachlorhydrate, monohydrate

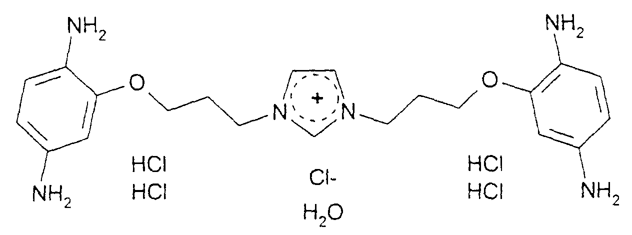

### a) Préparation du N-[2-(3-imidazol-1-yl-propoxy)-4-nitro-phényl]-acétamide

On a chauffé au bain-marie bouillant pendant 4 heures 54,5 g (0,2 mole) de N-[2-(3-chloro-propoxy)-4-nitro-phényl]-acétamide, dont la préparation a été décrite à l'étape a) de l'exemple 5 ci-dessus, et 40,8 g (0,6 mole) de 1H-imidazole dans 150 ml de diméthylformamide.

On a versé sur 900 g d'eau glacée, essoré le précipité cristallisé, lavé à l'eau et recristallisé de l'isopropanol au reflux.

Après séchage à 45°C sous vide on a obtenu 30,2 g de cristaux jaune pâle qui ont fondu à 139°C (Kofler) et dont l'analyse élémentaire était conforme à celle calculée pour C₁₄H₁₆N₄O₄.

### b) Préparation du chlorure de 1,3-bis-[3-(2-acétylamino-5-nitro-phénoxy)-propyl]-3H-imidazol-1-ium

On a utilisé le mode opératoire décrit ci-dessus pour l'exemple 5, étape b).

A partir de 18,9 g (0,062 mole) de N-[2-(3-imidazol-1-yl-propoxy)-4-nitro-phényl]-acétamide obtenu à l'étape précédente et de 18,6 g (0,068 mole) de N-[2-(3-chloro-propoxy)-4-nitro-phényl]-acétamide dont la préparation a été décrite à l'étape a) de l'exemple 6 ci-dessus, on a obtenu, après recristallisation d'un mélange eau/éthanol au reflux, 28,2 g de cristaux jaune pâle de chlorure de 1,3-bis-[3-(2-acétylamino-5-nitro-phénoxy)-propyl]-3H-imidazol-1-ium qui ont fondu à 190°C (Kofler) et dont l'analyse élémentaire était conforme à celle calculée pour C₂₅H₂₉N₆O₈Cl.

### c) Réduction du chlorure de 1,3-bis-[3-(2-acétylamino-5-nitro-phénoxy)-propyl]-3H-imidazol-1-ium

On a utilisé le mode opératoire décrit pour l'exemple 1, étape c), sans salifier le composé réduit.

A partir de 28,0 g (0,0485 mole) de chlorure de 1,3-bis-[3-(2-acétylamino-5-nitro-phénoxy)-propyl]-3H-imidazol-1-ium synthétisé ci-dessus à l'étape précédente, on a obtenu 23,5 g de cristaux blancs de chlorure de 1,3-bis-[3-(2-acétylamino-5-amino-phénoxy)-propyl]-3H-imidazol-1-ium dont l'analyse élémentaire était conforme à celle calculée pour C₂₅H₃₃N₆O₄Cl.

### d) Désacétylation du chlorure de 1,3-bis-[3-(2-acétylamino-5-amino-phénoxy)-propyl]-3H-imidazol-1-ium

On utilise le mode opératoire décrit pour l'exemple 5, étape c).

A partir de 23,5 g (0,0454 mole) de chlorure de 1,3-bis-[3-(2-acétylamino-5-amino-phénoxy)-propyl]-3H-imidazol-1-ium synthétisé ci-dessus à l'étape précédente, on a obtenu 15,0 g de cristaux blancs qui ont fondu avec décomposition à 210-215°C (Kofler), dont la RMN 1H était conforme et dont l'analyse élémentaire calculée pour C₂₁H₃₃N₆O₂Cl₅ + H₂O était :

| % | C | H | N | O | Cl |
|---|---|---|---|---|---|
| Calculé | 42,26 | 5,91 | 14,08 | 8,04 | 29,70 |
| Trouvé | 41,89 | 6,03 | 13,98 | 9,32 | 30,11 |

### EXEMPLE DE PREPARATION 7 : Synthèse du dichlorure de 1,4-bis-4-[3-(5-amino-2-hydroxy-benzyl)-3H-imidazol-1-ium]-butane, dichlorhydrate, monohydrate

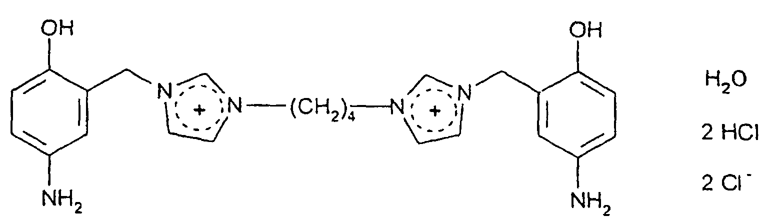

### a) Préparation du dichlorure de 1,4-bis-4-[3-(5-nitro-2-hydroxy-benzyl)-3H-imidazol-1-ium]-butane, monohydrate

On a chauffé pendant 8 heures au bain-marie bouillant 37,5 g (0,2 mole) de 2-chlorométhyl-4-nitro-phénol et 19,0 g (0,1 mole) de 1,4-di-imidazol-1-yl-butane dans 200 ml de toluène.

La gomme en suspension a été décantée et reprise dans l'éthanol absolu jusqu'à cristallisation complète.

Après essorage et recristallisation d'un mélange éthanol/eau au reflux on a obtenu 31,3 g de cristaux jaunes qui ont fondu à une température supérieure à 260°C (Kofler) et dont l'analyse élémentaire calculée pour C₂₄H₂₆N₆O₆Cl₂ + H₂O était :

| % | C | H | N | O | Cl |
|---|---|---|---|---|---|
| Calculé | 49,41 | 4,84 | 14,40 | 19,20 | 12,15 |
| Trouvé | 49,55 | 4,80 | 14,18 | 19,76 | 12,10 |

### b) Réduction du dichlorure de 1,4-bis-4-[3-(5-nitro-2-hydroxy-benzyl)-3H-imidazol-1-ium]-butane, monohydrate

On a utilisé le mode opératoire décrit pour l'exemple 1, étape c).

A partir de 31,0 g (0,055 mole) de dichlorure de 1,4-bis-[3-(5-nitro-2-hydroxy-benzyl)-3H-imidazol-1-ium]-butane, monohydrate synthétisés ci-dessus à l'étape précédente on a obtenu 24,8 g de cristaux blancs qui ont fondu à une température supérieure à 260°C (Kofler), dont la structure était conforme en RMN 1H

### EXEMPLE DE PREPARATION 8 : Synthèse du dichlorure de 1,3-bis-{3-[3-(2,5-diamino-phénoxy)-propyl]-3H-imidazol-1-ium}-propane, tétrachlorhydrate, dihydrate

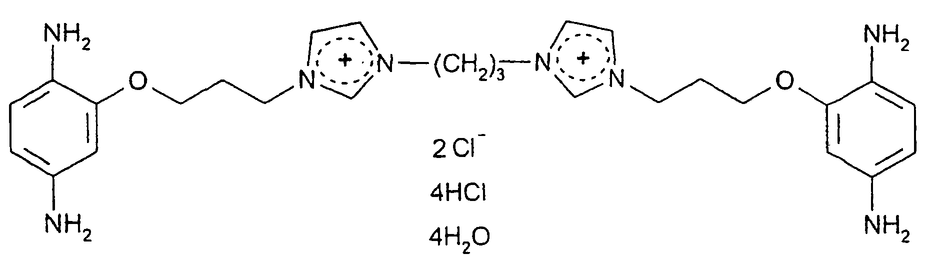

### a) Préparation du dichlorure de 1,3-bis-{3-[3-(2-acétylamino-5-nitro-phénoxy)-propyl]-3H-imidazol-1-ium}-propane + 1,5 H₂O

On a chauffé pendant 10 heures au reflux un mélange de 27,4 g (0,09 mole) de N-[2-(3-imidazol-1-yl-propoxy)-4-nitro-phényl]-acétamide dont la préparation a été décrite ci-dessus à l'exemple 6, étape a) et de 5,1g (0,045 mole) de 1,3-dichloro-propane dans 60 ml de pentanol-1.

On a refroidi à température ambiante, essoré le précipité cristallisé, lavé à l'éthanol absolu et recristallisé de l'éthanol à 96° au reflux.

On a obtenu 23,7 g de cristaux jaune pâle de dichlorure de 1,3-bis-{3-[3-(2-acétylamino-5-nitro-phénoxy)-propyl]-3H-imidazol-1-ium}-propane + 1,5 H₂O qui ont fondu à 187-188°C et dont l'analyse élémentaire calculée pour C₃₁H₃₈N₈O₈Cl₂ +1,5 H₂O est :

| % | C | H | N | O | Cl |
|---|---|---|---|---|---|
| Calculé | 49,74 | 5,52 | 14,97 | 20,30 | 9,47 |
| Trouvé | 49,76 | 5,61 | 14,93 | 20,30 | 9,71 |

### b) Réduction et désacétylation du dichlorure de 1,3-bis-4-{3-[3-(2-acétylamino-5-nitro-phénoxy)-propyl]-3H-imidazol-1-ium}-propane + 1,5 H₂O

Dans un hydrogénateur, on a placé 25,3 g (0,0338 mole) de dichlorure de 1,3-bis-{3-[3-(2-acétylamino-5-nitro-phénoxy)-propyl]-3H-imidazol-1-ium}-propane + 1,5 H₂O obtenu ci-dessus à l'étape précédente, 16 g de palladium à 5% sur charbon (contenant 50% d'eau), 300 ml d'éthanol et 300 ml d'eau.

La réduction s'est faite en une heure sous une pression d'hydrogène d'environ 8 bars et à une température qui a été portée progressivement à 80°C.

On a obtenu 22,2 g d'une laque de dichlorure de 1,3-bis-{3-[3-(2-acétylamino-5-amino-phénoxy)-propyl]-3H-imidazol-1-ium}-propane qui a été chauffée pendant ½ heure au bain-marie bouillant dans 42 ml d'acide chlorhydrique aqueux à 36%.

On a refroidi dans un bain de glace et dilué avec 100 ml d'éthanol absolu.

Le précipité cristallisé a été essoré, lavé à l'éthanol absolu et séché sous vide à 50°C sur anhydride phosphorique.

On a obtenu 19,0 g de cristaux blancs de dichlorure de 1,3-bis-{3-[3-(2,5-diamino-phénoxy)-propyl]-3H-imidazol-1-ium}-propane, tétrachlorhydrate, dihydrate qui ont fondu avec décomposition à 218-220°C et dont l'analyse élémentaire calculée pour C₂₇H₄₂N₈O₂Cl₆ +2 H₂O était :

| % | C | H | N | O | Cl |
|---|---|---|---|---|---|
| Calculé | 42,70 | 6,11 | 14,75 | 8,43 | 28,01 |
| Trouvé | 43,19 | 6,12 | 14,76 | 8,42 | 28,56 |

### EXEMPLES D'APPLICATION

### EXEMPLES 1 à 11 DE TEINTURE EN MILIEU BASIQUE

On a préparé les compositions tinctoriales suivantes (teneurs en grammes; les composants 4 et 5 ne font pas partie de l'invention):

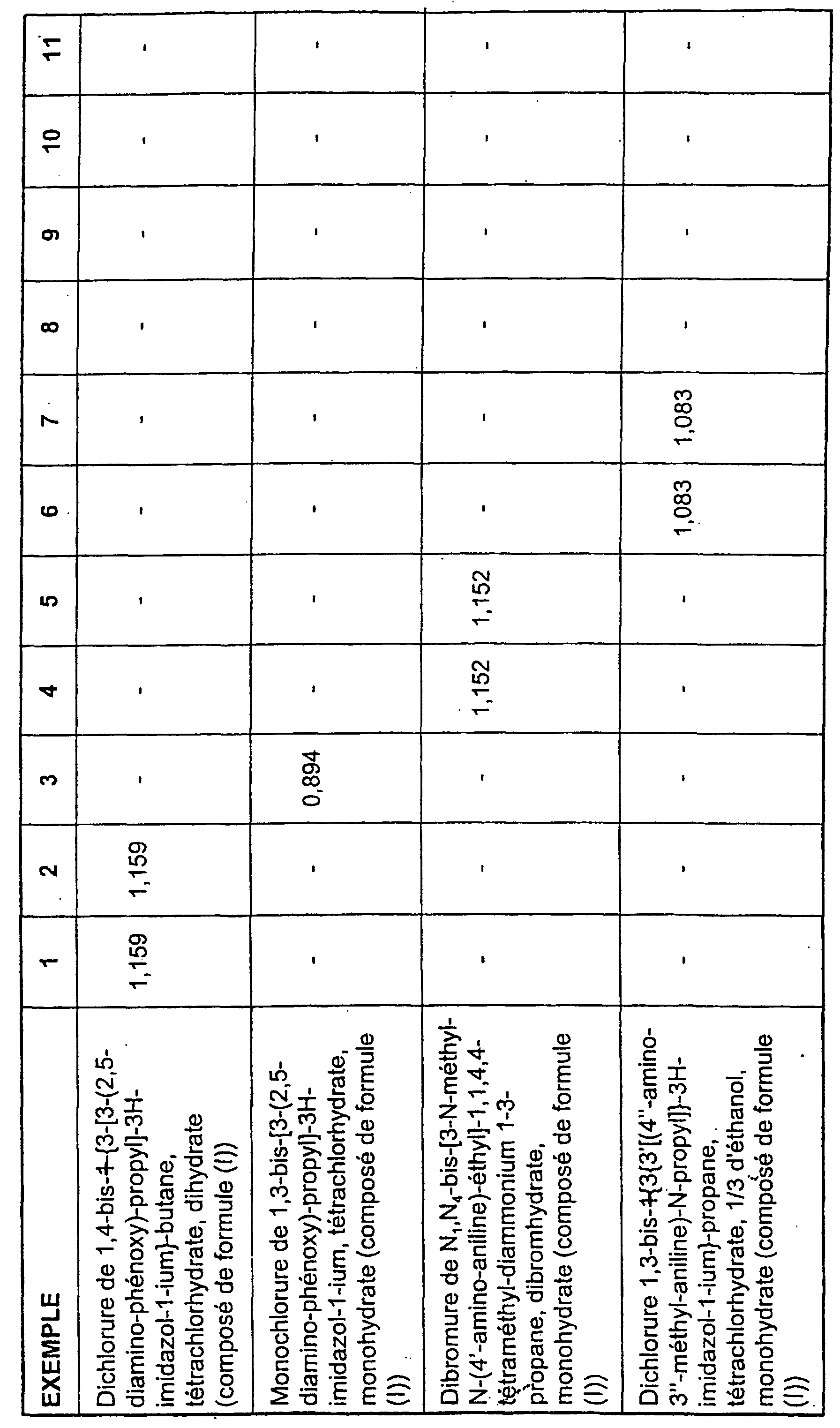

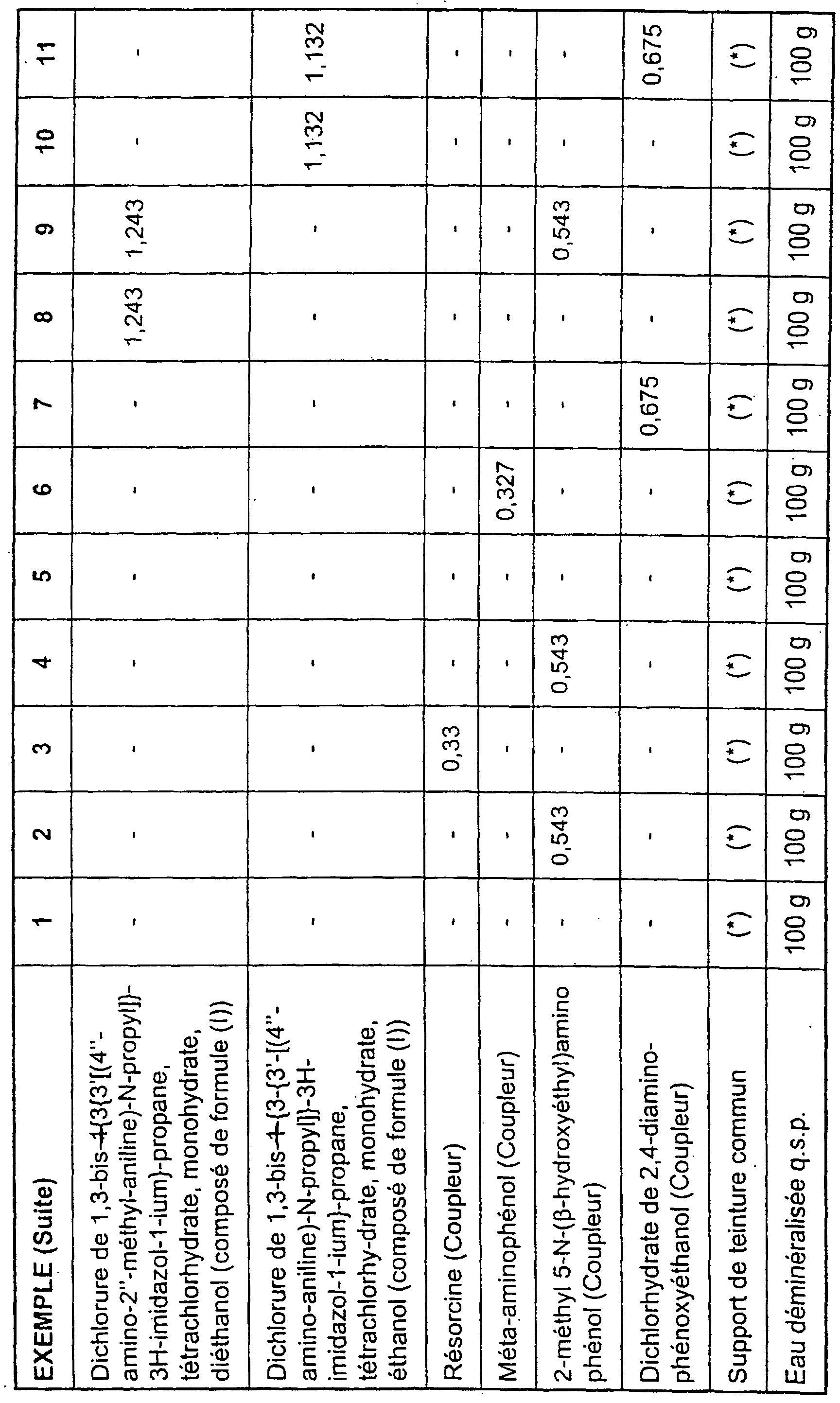

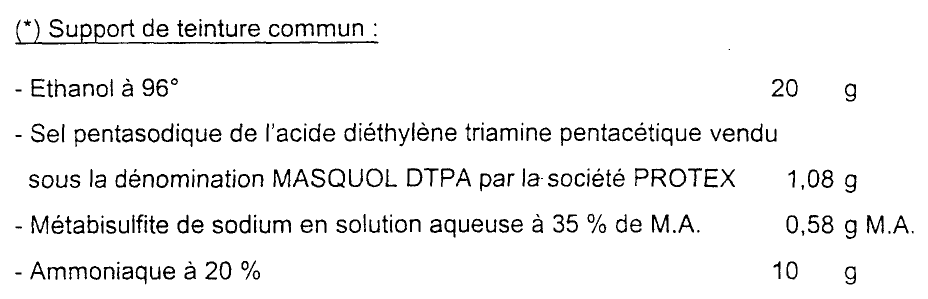

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris, naturels ou permanentés, à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de TEINTURE** | **Nuance sur cheveux naturels** | **Nuance sur cheveux permanentés** |
|---|---|---|---|
| **1** | 10 ± 0,2 | Blond foncé naturel cendré doré | Blond clair cendré |
| **2** | 10 ± 0,2 | Bleu | Châtain violine |
| **3** | 10 ± 0,2 | Châtain cendré légèrement mat | Châtain clair naturel cendré doré |
| **4** | 10 ± 0,2 | Bleu | Châtain clair violine irisé |
| **5** | 10 ± 0,2 | Châtain clair mat | Châtain cendré violacé |
| **6** | 10 ± 0,2 | Gris mat | Gris foncé |
| **7** | 10 ± 0,2 | Bleu vert rabattu | Bleu vert |
| **8** | 10 ± 0,2 | Irisé acajou | Acajou |
| **9** | 10 ± 0,2 | Violine irisé | Violine irisé |
| **10** | 10 ± 0,2 | Blond foncé doré cendré | Châtain clair cendré |
| **11** | 10 ± 0,2 | Bleu | Bleu |

## Revendications

1. Composés de formule (I) suivante, et leurs sels d'addition avec un acide : 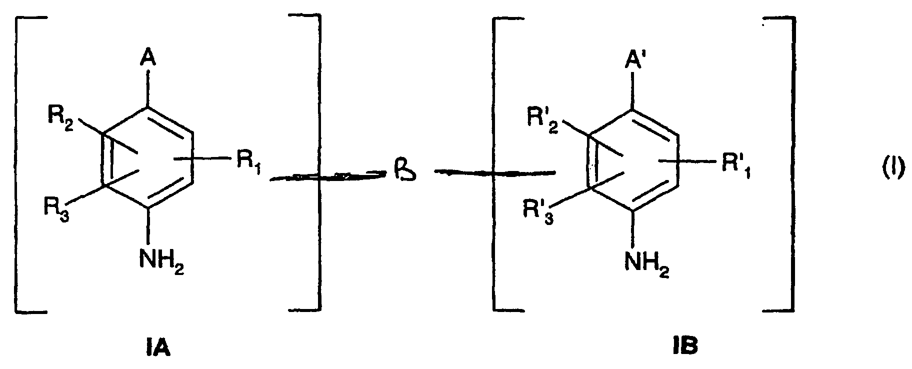 dans laquelle :
• R₁, R₂, R₃, R'₁, R'₂ et R'₃, qui peuvent être identiques ou différents, représentent un atome d'hydrogène ; un atome d'halogène ; un radical alkyl(C₁-C₆) carbonyle ; un radical aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; ou un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, alkyl(C₁-C₆) carboxy, carbamyle, N-alkyl(C₁-C₆)carbamyle, N,N-dialkyl(C₁-C₆)carbamyle, alkyl(C₁-C₆)sulfonyle, aminosulfonyle, N-alkyl(C₁-C₆)aminosulfonyle, N,N-dialkyl(C₁-C₆)aminosulfonyle, thiocarbamyle, formyle ;
• R₁ et R'₁ peuvent former ensemble un bras de liaison B,
• R₆ désigne un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle ;
• A représente un groupement -NR₄R₅ ou un radical hydroxyle ;
• A' représente un groupement -NR'₄R'₅ ou un radical hydroxyle ;
• R₄, R₅, R'₄ et R'₅, identiques ou différents, représentent un atome d'hydrogène ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle ;
• R₄ avec R'₄ peuvent former ensemble un bras de liaison B ;
• Z est choisi parmi les cycles pyrrolique, imidazolique, pyrazolique, oxazolique, thiazolique et triazolique, pyridinium, pyrimidinium, pyrazinium, oxazinium, triazinium, pyrrolidinium, pipéridinium, pipérazinum ou morpholinium
• B est un bras de liaison qui relie AI à AII et qui représente une chaîne alkylène comportant de préférence de 1 à 14 atomes de carbone, linéaire ou ramifiée pouvant être interrompue par un ou plusieurs groupements Z et/ou par un ou plusieurs hétéroatomes choisis parmi les atomes d'oxygène, de soufre ou d'azote, et éventuellement substituée par un ou plusieurs radicaux hydroxyle ou alcoxy en C₁-C₆, et pouvant porter une ou plusieurs fonctions cétone ;
étant entendu :
- que le nombre de groupement cationique Z est au moins égal à 1 ;
- qu'il existe un seul bras de liaison entre AI et AII.

2. Composés selon l'une quelconque des revendications précédentes, **caractérisés par le fait qu'**ils sont choisis parmi :
- le dichlorure de 1,3-bis-{3{3'[(4"-amino-3"-méthyl-aniline)-N-propyl]}-3H-imidazol-1-ium}-propane ;
- le dichlorure de 1,3-bis-{3{3'[(4"-amino-2"-méthyl-aniline)-N-propyl]}-3H-imidazol-1-ium}-propane, monohydrate, diéthanol ;
- le dichlorure de 1,3-bis-{3{3'[(4"-amino-aniline)-N-propyl]}-3H-imidazol-1-ium}-propane, monohydrate, éthanol ;
- le dichlorure de 1,3-bis-{3{3'[(4"-amino-aniline)-N-propyl]}-3H-imidazol-1-ium}-2-propanol, monohydrate ;
- le dichlorure de 1,4-bis-{3[3-(2,5-diamino-phénoxy)-propyl]-3H-imidazol-1-ium}-butane, dihydrate ;
- le monochlorure de 1,3-bis-[3-(2,5-diamino-phénoxy)-propyl]-3H-imidazol-1-ium, monohydrate ;
- le dichlorure de 1,4-bis-[3-(5-amino-2-hydroxy-benzyl)-3H-imidazol-1ium]-butane, monohydrate ;
- le dichlorure de 1,3-bis-{3-[3-(2,5-diamino-phénoxy)-propyl]-3H-imidazol-1-ium}-propane, dihydrate ;
- le dichlorure de 1,3-bis-{3{3'[(4"-amino-aniline)-N-propyl]}-3H-imidazol-1-ium}-propane ;
- le dichlorure de 1,3-bis-{4{4'(4-[3-(4"-amino-phénylamino)-propyl]}-1,3-diméthyl-3H-imidazol-1-ium}-propane,
- le dichlorure de 1,3-bis-{4{4'(4-[3-(4"-amino-2"-méthyl-aniline)-propyl]}-1,3-diméthyl-3H-imidazol-1-ium}-propane ;
- le monochlorure de [2-(2,5-diamino-phénoxy)-éthyl]-3-[3-(2,5-diamino-phénoxy)-propyl]-1-méthyl-3-imidazol-1-ium ;
- le monochlorure de [2-(2,5-diamino-phénoxy)-éthyl]-1-[3-(2,5-diamino-phénoxy)-propyl]-3-méthyl-3-imidazol-1-ium ;
et leurs sels d'addition avec un acide.

3. Composés selon la revendication 2, **caractérisés par le fait qu'**ils sont choisis parmi:
- le dichlorure de 1,3-bis-{3-{3'-[(4"-amino-aniline)-N-propyl]}-3H-imidazol-1-ium}-propane, monohydrate, éthanol ;
- le monochlorure de 1,3-bis-[3-(2,5-diamino-phénoxy)-propyl]-3H-imidazol-1-ium, monohydrate ;
- le dichlorure de 1,4-bis-[3-(5-amino-2-hydroxy-benzyl)-3H-imidazol-1-ium]-butane, monohydrate ;
- le dichlorure de 1,3-bis-{3{3'[(4"-amino-aniline)-N-propyl]}-3H-imidazol-1-ium}-propane ;
et leurs sels d'addition avec un acide.

4. Utilisation des composés de formule (I) tels que définis à l'une quelconque des revendications précédentes, à titre de base d'oxydation pour la teinture d'oxydation des fibres kératiniques, et en particulier des fibres humaines telles que les cheveux.

5. Composition pour la teinture d'oxydation des fibres kératiniques et en particulier des fibres kératiniques humaines telles que les cheveux, **caractérisée par le fait qu'**elle comprend, dans un milieu approprié pour la teinture, au moins un composé de formule (I) tel que défini à l'une quelconque des revendications 1 à 3, à titre de base d'oxydation.

6. Composition selon la revendication 5, **caractérisée par le fait que** le ou les composés de formule (I) représentent de 0,0005 à 12 % en poids du poids total de la composition tinctoriale.

7. Composition selon la revendication 6, **caractérisée par le fait que** le ou les composés de formule (I) représentent de 0,005 à 6 % en poids du poids total de la composition tinctoriale.

8. Composition selon l'une quelconque des revendications 5 à 7, **caractérisée par le fait que** le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

9. Composition selon l'une quelconque des revendications 5 à 8, **caractérisée par le fait qu'**elle présente un pH compris entre 3 et 12.

10. Composition selon l'une quelconque des revendications 5 à 9, **caractérisée par le fait qu'**elle renferme au moins une base d'oxydation additionnelle choisie parmi les paraphénylènediamines, les bis-phénylalkylènediamines différentes des composés de formule (I), les para-aminophénols, les ortho-aminophénols et les bases hétérocycliques.

11. Composition selon la revendication 10, **caractérisée par le fait que** la ou les bases d'oxydation additionnelles représentent de 0,0005 à 12 % en poids du poids total de la composition tinctoriale.

12. Composition selon l'une quelconque des revendications 5 à 11, **caractérisée par le fait qu'**elle renferme au moins un coupleur et/ou au moins un colorant direct.

13. Composition selon la revendication 12, **caractérisée par le fait que** le ou les coupleurs sont choisis parmi les métaphénylènediamines, les méta-aminophénols, les métadiphénols et les coupleurs hétérocycliques, et leurs sels d'addition avec un acide.

14. Composition selon la revendication 12 ou 13, **caractérisée par le fait que** le ou les coupleurs représentent de 0,0001 à 10 % en poids du poids total de la composition tinctoriale.

15. Composition selon l'une quelconque des revendications 5 à 14, **caractérisée par le fait que** les sels d'addition avec un acide sont choisis parmi les chlorhydrates, les bromhydrates, les sulfates, les citrates, les succinates, les tartrates, les lactates et les acétates.

16. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 5 à 15, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

17. Procédé selon la revendication 16, **caractérisé par le fait que** l'agent oxydant est choisi parmi le peroxyde d'hydrogène, le peroxyde d'urée, les bromates de métaux alcalins, les persels tels que les perborates et persulfates.

18. Dispositif à plusieurs compartiments, ou "kit" de teinture à plusieurs compartiments, dont un premier compartiment renferme une composition tinctoriale telle que définie à l'une quelconque des revendications 5 à15 et un second compartiment renferme une composition oxydante.

## Patentansprüche

1. Verbindungen der folgenden Formel (I) und ihre Additionssalze mit einer Säure: 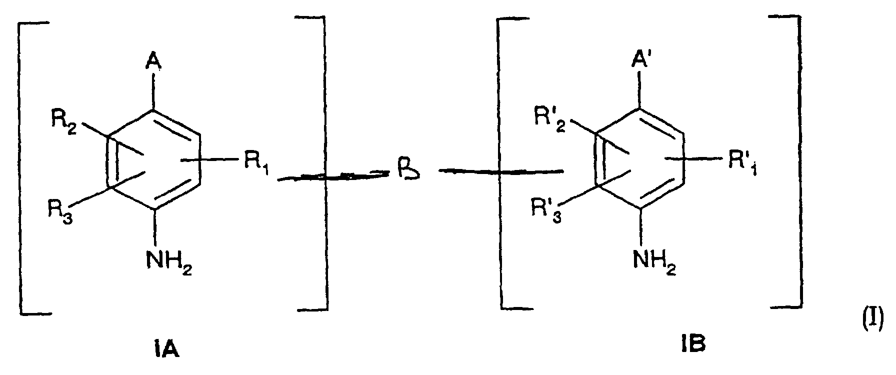 wobei in der Formel bedeuten:
- die Gruppen R₁, R₂, R₃, R'₁, R'₂ R'₃, die gleich oder verschieden sein können, bedeuten Wasserstoff; Halogen; Alkyl(C₁₋₆)carbonyl; Aminoalkyl(C₁₋₆)carbonyl; N-Alkyl(C₁₋₆)aminoalkyl(C₁₋₆)carbonyl; N,N-Dialkyl(C₁₋₆)aminoalkyl(C₁₋₆)carbonyl; Aminoalkyl(C₁₋₆)carbonylalkyl(C₁₋₆); N-Alkyl(C₁₋₆)aminoalkyl(C₁₋₆)carbonylalkyl(C₁₋₆); N,N-Dialkyl(C₁₋₆)aminoalkyl(C₁₋₆)carbonylalkyl(C₁₋₆); Carboxy; Alkyl(C₁₋ ₆)carboxy; Alkyl(C₁₋₆)sulfonyl; Aminosulfonyl; N-Alkyl(C₁₋₆)aminosulfonyl; N,N-Dialkyl(C₁₋₆)aminosulfonyl; Aminosulfonylalkyl(C₁₋ ₆); N-Alkyl(C₁₋₆)amino-sulfonylalkyl(C₁₋₆); N,N-Dialkyl(C₁₋₆)aminosulfonylalkyl(C₁₋₆); Carbamoyl; N-Alkyl(C₁₋₆)carbamoyl; N,N-Dialkyl(C₁₋₆)carbamoyl; Carbamoylalkyl(C₁₋₆); N-Alkyl(C₁₋₆)-carbamoylalkyl(C₁₋₆); N,N-Dialkyl(C₁₋₆)-carbamoylalkyl(C₁₋₆); C₁₋₆-Alkyl; C₁₋₆-Monohydroxyalkyl; C₂₋₆-Polyhydroxyalkyl; Alkoxy(C₁₋₆)-alkyl(C₁₋₆); C₁₋₆-Trifluoralkyl; Cyano; OR₆ oder SR₆; eine Aminogruppe, die mit Alkyl(C₁₋₆)carbonyl, Alkyl(C₁₋₆)carboxy, Trifluoralkyl(C₁₋₆)carbonyl, Aminoalkyl(C₁₋₆)carbonyl, N-Alkyl(C₁₋₆)-aminoalkyl(C₁₋₆)carbonyl, N,N-Dialkyl(C₁₋₆)aminoalkyl(C₁₋₆)carbonyl, Alkyl(C₁₋₆)carboxy, Carbamoyl, N-Alkyl(C₁₋₆)carbamoyl, N,N-Dialkyl-(C₁₋₆)carbamoyl, Alkyl(C₁₋₆)sulfonyl, Aminosulfonyl, N-Alkyl(C₁₋₆)-aminosulfonyl, N,N-Dialkyl(C₁₋₆)-aminosulfonyl, Thiocarbamoyl oder Formyl geschützt ist;
- wobei R₁ und R'₁ gemeinsam eine Verbindungsgruppe B bilden können;
- die Gruppe R₆ bedeutet C₁₋₆-Alkyl; C₁₋₆-Monohydroxyalkyl; C₂₋₆-Polyhydroxyalkyl; Alkoxy(C₁₋₆)alkyl(C₁₋₆); Aryl; Benzyl; C₁₋₆-Carboxyalkyl; Alkyl(C₁₋₆)carboxyalkyl(C₁₋₆); C₁₋₆-Cyanoalkyl; C₁₋₆-Carbamoylalkyl; N-Alkyl(C₁₋₆)carbamoylalkyl(C₁₋₆); N,N-Dialkyl(C₁₋ ₆)carbamoylalkyl(C₁₋₆); C₁₋₆-Trifluoralkyl; C₁₋₆-Aminosulfonylalkyl; N-Alkyl(C₁₋₆)aminosulfonylalkyl(C₁₋₆); N,N-Dialkyl(C₁₋₆)aminosulfonylalkyl(C₁₋₆); Alkyl(C₁₋₆)sulfinylalkyl(C₁₋₆); Alkyl(C₁₋₆)-sulfonylalkyl(C₁₋₆); Alkyl(C₁₋₆)carbonylalkyl(C₁₋₆); C₁₋₆-Aminoalkyl; C₁₋₆-Aminoalkyl, wobei die Aminogruppe mit einer oder zwei Gruppen substituiert ist, die identisch oder voneinander verschieden sind und unter den folgenden Gruppen ausgewählt sind: C₁₋₆-Alkyl, C₁₋₆-Monohydroxyalkyl, C₂₋₆-Polyhydroxyalkyl, Alkyl(C₁₋₆)carbonyl, Formyl, Trifluoralkyl(C₁₋₆)carbonyl, Alkyl(C₁₋ ₆)-carboxy, Carbamoyl, N-Alkyl(C₁₋₆)carbamoyl, N,N-Dialkyl(C₁₋₆)-carbamoyl, Thiocarbamoyl oder Alkyl(C₁₋₆)sulfonyl;
- die Gruppe A bedeutet eine Gruppe -NR₄R₅ oder Hydroxy;
- die Gruppe A' bedeutet eine Gruppe -NR'₄R'₅ oder Hydroxy;
- die Gruppen R₄, R'₄, R₅ und R'₅, die gleich oder verschieden sind, bedeuten Wasserstoff; C₁₋₆-Alkyl; C₁₋₆-Monohydroxyalkyl; C₂₋₆-Polyhydroxyalkyl; Alkoxy(C₁₋₆)alkyl(C₁₋₆); Aryl; Benzyl; C₁₋₆-Cyanoalkyl; C₁₋₆-Carbamoylalkyl; N-Alkyl(C₁₋₆)carbamoylalkyl(C₁₋₆); N,N-Dialkyl(C₁₋₆)carbanloylalkyl(C₁₋₆); C₁₋₆-Thiocarbamoylalkyl; C₁₋₆-Trifluoralkyl; C₁₋₆-Sulfoalkyl; Alkyl(C₁₋₆)carboxyalkyl(C₁₋₆); Alkyl-(C₁₋₆)sulfinylalkyl(C₁₋₆); C₁₋₆-Aminosulfonylalkyl; N-Alkyl(C₁₋₆)-aminosulfonylalkyl(C₁₋₆); N,N-Dialkyl(C₁₋₆)aminosulfonylalkyl(C₁₋ ₆); Alkyl(C₁₋₆)carbonylalkyl(C₁₋₆); C₁₋₆-Aminoalkyl; C₁₋₆-Aminoalkyl, dessen Aminogruppe mit einer oder zwei Gruppen substituiert ist, die identisch oder voneinander verschieden sind und unter den folgenden Gruppen ausgewählt sind: Alkyl, C₁₋₆-Monohydroxyalkyl, C₂₋₆-Polyhydroxyalkyl, Alkyl(C₁₋₆)carbonyl, Carbamoyl, N-Alkyl(C₁₋₆)carbamoyl, N,N-Dialkyl(C₁₋₆)carbamoyl, Alkyl(C₁₋₆)sulfonyl, Formyl, Trifluoralkyl(C₁₋₆)carbonyl, Alkyl(C₁₋₆)-carboxy, Thiocarbamoyl;
- wobei R₄ und R'₄ gemeinsam eine Verbindungsgruppe B bilden können;
- Z ist unter den Ringen Pyrrol, Imidazol, Pyrazol, Oxazol, Thiazol, Triazol, Pyridinium, Pyrimidinium, Pyrazinium, Oxazinium, Triazinium, Pyrrolidinium, Piperidinium, Piperazinium oder Morpholinium ausgewählt;
- B ist eine Verbindungsgruppe, die AI mit AII verbindet und die eine geradkettige oder verzweigte Alkylengruppe bedeutet, welche vorzugsweise 1 bis 14 Kohlenstoffatome aufweist und gegebenenfalls mit einer oder mehreren Gruppen Z und/oder einem oder mehreren Heteroatomen, wie Sauerstoff, Schwefel oder Stickstoff unterbrochen sein kann, gegebenenfalls mit einer oder mehreren Hydroxygruppen oder C₁₋₆-Alkoxygruppen substituiert sein kann und eine oder mehrere Ketogruppen enthalten kann;
mit der Maßgabe, dass:
- die Anzahl der kationischen Gruppen Z mindestens 1 ist; und
- nur eine Verbindungsgruppe zur Verknüpfung von AI und AII existiert.

2. Verbindungen nach einem der vorhergehenden Ansprüche, **dadurch gekennzeichnet, dass** sie ausgewählt sind unter:
- 1,3-Bis-{3{3'[(4"-amino-3"-methylanilin)-N-propyl]}-3H-imidazol-1-ium}-propan-dichlorid;
- 1,3-Bis-{3{3'[(4"-amino-2"-methylanilin)-N-propyl]}-3H-imidazol-1-ium}-propan-dichlorid, Monohydrat, Diethanol;
- 1,3-Bis-{3{3'[(4"-aminoanilin)-N-propyl]}-3H-imidazol-1-ium}-propan-dichlorid, Monohydrat, Ethanol;
- 1,3-Bis-{3{3'[(4"-aminoanilin)-N-propyl]}-3H-imidazol-1-ium}-2-propanol-dichlorid, Monohydrat;
- 1,4-Bis-{3[3(2,5-diaminophenoxy)-propyl]-3H-imidazol-1-ium}-butan-dichlorid, Dihydrat;
- 1,3-Bis-[3-(2,5-diaminophenoxy)-propyl]-3H-imidazol-1-iummonochlorid, Monohydrat;
- 1,4-Bis-[3-(5-amino-2-hydroxy-benzyl)-3H-imidazol-1-ium]-butan-dichlorid, Monohydrat;
- 1,3-Bis-{3-[3-(2,5-diaminophenoxy)-propyl]-3H-imidazol-1-ium}-propan-dichlorid, Dihydrat;
- 1,3-Bis-{3{3'[(4"-amino-anilin)-N-propyl]}-3H-imidazol-1-ium}-propan-dichlorid;
- 1,3-Bis-{4{4'(4-[3-(4"-amino-phenylamino)-propyl]}-1,3-dimethyl-3H-imidazol-1-ium}-propan-dichlorid;
- 1,3-Bis-{4{4'(4-[3-(4"-amino-2"-methyl-anilin)-propyl]}-1,3-dimethyl-3H-imidazol-1-ium}-propan-dichlorid;
- 4-[2-(2,5-Diaminophenoxy)-ethyl]-3-[3-(2,5-diaminophenoxy)-propyl]-1-methyl-3-imidazol-1-ium-monochlorid;
- 4-[2-(2,5-Diaminophenoxy)-ethyl]-1-[3-(2,5-diaminophenoxy)-propyl]-3-methyl-3-imidazol-1-ium-monochlorid;
- und den Additionssalzen dieser Verbindungen mit einer Säure.

3. Verbindungen nach 2, **dadurch gekennzeichnet, dass** sie ausgewählt sind unter:
- 1,3-Bis-{3-{3'-[(4"-aminoanilin)-N-propyl]}-3H-imidazol-1-ium}-propan-dichlorid; Monohydrat, Ethanol;
- 1,3-Bis-[3-(2,5-diaminophenoxy)-propyl]-3H-imidazol-1-iummonochlorid, Monohydrat;
- 1,4-Bis-[3-(5-amino-2-hydroxy-benzyl)-3H-imidazol-1-ium]-butan-dichlorid, Monohydrat;
- 1,3-Bis-{3{3'[(4"-aminoanilin)-N-propyl]}-3H-imidazol-1-ium}-propan-dichlorid;
- und den Additionssalzen dieser Verbindungen mit einer Säure.

4. Verwendung von Verbindungen der Formel (I) nach einem der vorhergehenden Ansprüche als Oxidationsbasen zum oxidativen Färben von Keratinfasern und insbesondere menschlichen Keratinfasern, wie dem Haar.

5. Zusammensetzung zum oxidativen Färben von Keratinfasern und insbesondere menschlichen Keratinfasern, wie dem Haar, **dadurch gekennzeichnet, dass** sie in einem zum Färben geeigneten Medium als Oxidationsbase mindestens eine Verbindung der Formel (I) nach einem der Ansprüche 1 bis 3 enthält.

6. Zusammensetzung nach Anspruch 5, **dadurch gekennzeichnet, dass** die Verbindung(en) der Formel (I) 0,0005 bis 12 Gew.-% des Gesamtgewichts der Farbmittelzusammensetzung ausmachen.

7. Zusammensetzung nach Anspruch 6, **dadurch gekennzeichnet, dass** die Verbindung(en) der Formel (I) 0,005 bis 6 Gew.-% des Gesamtgewichts der Farbmittelzusammensetzung ausmachen.

8. Zusammensetzung nach einem der Ansprüche 5 bis 7, **dadurch gekennzeichnet, dass** das zum Färben geeignete Medium (oder der Träger) aus Wasser oder einem Gemisch von Wasser und mindestens einem organischen Lösungsmittel besteht, das unter den niederen C₁₋₄-Alkanolen, Glycerin, Glykolen und Glykolethern, aromatischen Alkoholen, analogen Produkten und deren Gemischen ausgewählt ist.

9. Zusammensetzung nach einem der Ansprüche 5 bis 8, **dadurch gekennzeichnet, dass** sie einen pH-Wert im Bereich von 3 bis 12 aufweist.

10. Zusammensetzung nach einem der Ansprüche 5 bis 9, **dadurch gekennzeichnet, dass** sie mindestens eine zusätzliche Oxidationsbase enthält, die unter den p-Phenylendiaminen, den Bisphenylalkylendiaminen, die von den Verbindungen der Formel (I) verschieden sind, den p-Aminophenolen, den o-Aminophenolen und den heterocyclischen Basen ausgewählt ist.

11. Zusammensetzung nach Anspruch 10, **dadurch gekennzeichnet, dass** die zusätzliche(n) Oxidationsbase(n) 0,0005 bis 12 Gew.-% des Gesamtgewichts der Farbmittelzusammensetzung ausmachen.

12. Zusammensetzung nach einem der Ansprüche 5 bis 11, **dadurch gekennzeichnet, dass** sie mindestens einen Kuppler und/oder mindestens einen Direktfarbstoff enthält.

13. Zusammensetzung nach Anspruch 12, **dadurch gekennzeichnet, dass** der oder die Kuppler unter den m-Phenylendiaminen, m-Aminophenolen, m-Dihydroxybenzolen und den heterocyclischen Kupplern und deren Additionssalzen mit einer Säure ausgewählt sind.

14. Zusammensetzung nach Anspruch 12 oder 13, **dadurch gekennzeichnet, dass** der oder die Kuppler 0,0001 bis 10 Gew.-% des Gesamtgewichts der Farbmittelzusammensetzung ausmachen.

15. Zusammensetzung nach einem der Ansprüche 5 bis 14, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

16. Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 5 bis 15 aufgebracht wird und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

17. Verfahren nach Anspruch 16, **dadurch gekennzeichnet, dass** das Oxidationsmittel unter Wasserstoffperoxid, Harnstoffperoxid, Alkalimetallbromaten und Salzen von Persäuren, wie Perboraten und Persulfaten, ausgewählt ist.

18. Vorrichtung mit mehreren Abteilungen oder "Kit" zum Färben mit mehreren Abteilungen, wobei eine erste Abteilung eine Farbmittelzusammensetzung nach einem der Ansprüche 5 bis 15 und eine zweite Abteilung eine oxidierende Zusammensetzung enthält.

## Claims

1. Compounds of formula (I) below, and the addition salts thereof with an acid: 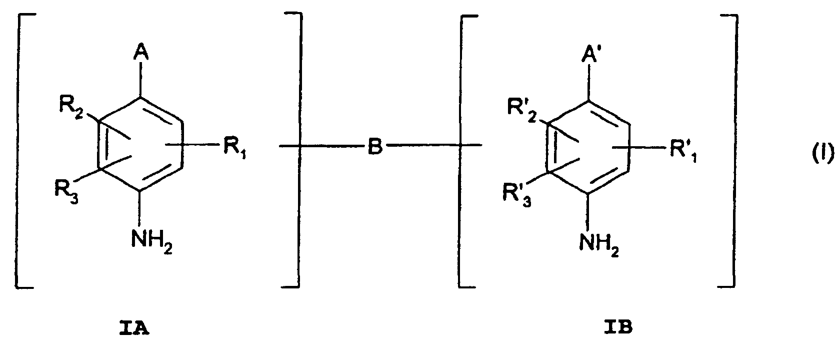 in which:
• R₁, R₂, R₃, R'₁, R'₂ and R'₃, which may be identical or different, represent a hydrogen atom; a halogen atom; a (C₁-C₆)alkylcarbonyl radical; an amino(C₁-C₆)alkylcarbonyl radical; an N-(C₁-C₆)alkylamino(C₁-C₆)alkylcarbonyl radical; an N,N-di (C₁-C₆)alkylamino(C₁-C₆)alkylcarbonyl radical; an amino(C₁-C₆)alkylcarbonyl(C₁-C₆)alkyl radical; an N- (C₁-C₆)alkylamino(C₁-C₆)alkylcarbonyl(C₁-C₆)alkyl radical; an N,N-di(C₁-C₆)alkylamino(C₁-C₆)-alkylcarbonyl(C₁-C₆)alkyl radical; a carboxyl radical; a (C₁-C₆)alkylcarboxyl radical; a C₁-C₆ alkylsulphonyl radical; an aminosulphonyl radical; a C₁-C₆ N-alkylaminosulphonyl radical; an N,N-di(C₁-C₆)alkylaminosulphonyl radical; a C₁-C₆ aminosulphonylalkyl radical; an N-(C₁-C₆)alkylaminosulphonyl(C₁-C₆)alkyl radical; an N,N-di(C₁-C₆)alkylaminosulphonyl(C₁-C₆)alkyl radical; a carbamyl radical; an N-(C₁-C₆)alkyl-carbamyl radical; an N,N-di(C₁-C₆)alkylcarbamyl radical; a carbamyl(C₁-C₆)alkyl radical; an N-(C₁-C₆)alkylcarbamyl(C₁-C₆)alkyl radical; an N, N-di (C₁-C₆)alkylcarbamyl(C₁-C₆)alkyl radical; a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; a C₁-C₆ trifluoroalkyl radical; a cyano radical; a group OR₆ or SR₆; or an amino group protected with a (C₁-C₆)alkylcarbonyl, (C₁-C₆)alkylcarboxyl, trifluoro (C₁-C₆)alkylcarbonyl, amino (C₁-C₆)alkyl-carbonyl, N-(C₁-C₆)alkylamino(C₁-C₆)alkylcarbonyl, N,N-di(C₁-C₆)alkylamino(C₁-C₆)alkylcarbonyl, (C₁-C₆)alkylcarboxyl, carbamyl, N-(C₁-C₆)alkylcarbamyl, N,N-di(C₁-C₆)alkylcarbamyl, C₁-C₆ alkylsulphonyl, aminosulphonyl, C₁-C₆ N-alkylaminosulphonyl, N,N-di(C₁-C₆)alkylaminosulphonyl, thiocarbamyl or formyl radical;
• R₁ and R'₁ may form together a linker arm B;
• R₆ denotes a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; an aryl radical; a benzyl radical; a carboxy(C₁-C₆)alkyl radical; a (C₁-C₆)alkylcarboxy(C₁-C₆)alkyl radical; a cyano(C₁-C₆)alkyl radical; a carbamyl(C₁-C₆)alkyl radical; an N-(C₁-C₆)alkylcarbamyl(C₁-C₆)alkyl radical; an N,N-di(C₁-C₆)alkylcarbamyl(C₁-C₆)alkyl radical; a C₁-C₆ trifluoroalkyl radical; a C₁-C₆ aminosulphonylalkyl radical; an N-(C₁-C₆)alkylaminosulphonyl(C₁-C₆)alkyl radical; an N, N-di (C₁-C₆)alkylaminosulphonyl(C₁-C₆)alkyl radical; a (C₁-C₆)alkylsulphinyl (C₁-C₆)alkyl radical; a (C₁-C₆)alkylsulphonyl (C₁-C₆)alkyl radical; a (C₁-C₆)alkylcarbonyl(C₁-C₆)alkyl radical; a C₁-C₆ aminoalkyl radical; a C₁-C₆ aminoalkyl radical in which the amine is substituted with one or two identical or different radicals chosen from C₁-C₆ alkyl, C₁-C₆ monohydroxyalkyl, C₂-C₆ polyhydroxyalkyl, (C₁-C₆)alkylcarbonyl, formyl, trifluoro(C₁-C₆)alkylcarbonyl, (C₁-C₆)alkylcarboxyl, carbamyl, N-(C₁-C₆)alkylcarbamyl, N,N-di(C₁-C₆)alkylcarbamyl, thiocarbamyl and C₁-C₆ alkylsulphonyl radicals,
• A represents a group -NR₄R₅ or a hydroxyl radical;
• A' represents a group -NR'₄R'₅ or a hydroxyl radical; .
• R₄, R₅, R'₄ and R'₅, which may be identical or different, represent a hydrogen atom; a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; an aryl radical; a benzyl radical; a cyano(C₁-C₆)alkyl radical; a carbamyl (C₁-C₆)alkyl radical; an N-(C₁-C₆)alkylcarbamyl(C₁-C₆)alkyl radical; an N,N-di(C₁-C₆)alkylcarbamyl(C₁-C₆)alkyl radical; a thiocarbamyl(C₁-C₆)alkyl radical; a C₁-C₆ trifluoroalkyl radical; a C₁-C₆ sulphoalkyl radical; a (C₁-C₆)alkylcarboxy(C₁-C₆)alkyl radical; a (C₁-C₆)alkylsulphinyl(C₁-C₆)alkyl radical; a C₁-C₆ aminosulphonylalkyl radical; an N-(C₁-C₆)alkylaminosulphonyl(C₁-C₆)alkyl radical; an N,N-di (C₁-C₆)alkylaminosulphonyl(C₁-C₆)alkyl radical; a (C₁-C₆)alkylcarbonyl(C₁-C₆)alkyl radical; a C₁-C₆ aminoalkyl radical; a C₁-C₆ aminoalkyl radical in which the amine is substituted with one or two identical or different radicals chosen from alkyl, C₁-C₆ monohydroxyalkyl, C₂-C₆ polyhydroxyalkyl, (C₁-C₆)alkylcarbonyl, carbamyl, N-(C₁-C₆)alkylcarbamyl, N,N-di(C₁-C₆)-alkylcarbamyl, C₁-C₆ alkylsulphonyl, formyl, trifluoro(C₁-C₆)alkylcarbonyl, (C₁-C₆)alkylcarboxyl and thiocarbamyl radicals;
• R₄ and R'₄ may form together a linker arm B;
• Z is chosen from the pyrrole, imidazole, pyrazole, oxazole, thiazole and triazole, pyridinium, pyrimidinium, pyrazinium, oxazinium, triazinium, pyrrolidinium, piperidinium, piperazinium or morpholinium rings;
• B is a linker arm which links AI to AII and which represents a linear or branched alkylene chain preferably containing from 1 to 14 carbon atoms, which can be interrupted by one or more groups Z and/or by one or more heteroatoms chosen from oxygen, sulphur or nitrogen atoms, and which can be optionally substituted with one or more hydroxyl or C₁-C₆ alkoxy radicals, and which can bear one or more ketone functions;
it being understood:
- that the number of cationic group Z is at least equal to 1;
- that only one linker arm exists between AI and AII.

2. Compounds according to any one of the preceding claims, **characterized in that** they are chosen from:
- 1,3-bis{3-{3'-[(4"-amino-3"-methylanilino)-N-propyl]}-3H-imidazol-1-ium}propane dichloride;
- 1,3-bis{3-{3'-[(4"-amino-2"-methylanilino)-N-propyl]}-3H-imidazol-1-ium}propane dichloride monohydrate diethanol;
- 1,3-bis{3-{3'-[(4"-aminoanilino)-N-propyl]}-3H-imidazol-1-ium}propane dichloride monohydrate ethanol;
- 1,3-bis{3-{3'-[(4"-aminoanilino)-N-propyl]}-3H-imidazol-1-ium}-2-propanol dichloride monohydrate;
- 1,4-bis{3-[3-(2,5-diaminophenoxy)propyl]-3H-imidazol-1-ium}butane dichloride dihydrate;
- 1,3-bis[3-(2,5-diaminophenoxy)propyl]-3H-imidazol-1-ium monochloride monohydrate;
- 1,4-bis[3-(5-amino-2-hydroxybenzyl)-3H-imidazol-1-ium]butane dichloride monohydrate;
- 1,3-bis{3-[3-(2,5-diaminophenoxy)propyl]-3H-imidazol-1-ium}propane dichloride dihydrate;
- 1,3-bis{3-{3'-[(4"-aminoanilino)-N-propyl]}-3H-imidazol-1-ium}propane dichloride;
- 1,3-bis{4-{4'-(4-[3-(4"-aminophenylamino)propyl}}-1,3-dimethyl-3H-imidazol-1-ium}propane dichloride,
- 1,3-bis{4-{4'-(4-[3-(4"-amino-2"-methylanilino)-propyl]}-1,3-dimethyl-3H-imidazol-1-ium}propane dichloride;
- 4-[2-(2,5-diaminophenoxy)ethyl]-3-[3-(2,5-diaminophenoxy)propyl]-1-methyl-3-imidazol-1-ium monochloride;
- 4-[2-(2,5-diaminophenoxy)ethyl]-1-[3-(2,5-diaminophenoxy)propyl]-3-methyl-3-imidazol-1-ium monochloride;
and the addition salts thereof with an acid.

3. Compounds according to Claim 2, **characterized in that** they are chosen from:
- 1,3-bis{3-{3'-[(4"-aminoanilino)-N-propyl]}-3H-imidazol-1-ium}propane dichloride monohydrate ethanol;
- 1,3-bis[3-(2,5-diaminophenoxy)propyl]-3H-imidazol-1-ium monochloride monohydrate;
- 1,4-bis[3-(5-amino-2-hydroxybenzyl)-3H-imidazol-1-ium]butane dichloride monohydrate;
- 1,3-bis{3-{3'-[(4"-aminoanilino)-N-propyl]}-3H-imidazol-1-ium}propane dichloride;
and the addition salts thereof with an acid.

4. Use of the compounds of formula (I) as defined in any one of the preceding claims, as oxidation bases for the oxidation dyeing of keratin fibres, and in particular of human fibres such as the hair.

5. Composition for the oxidation dyeing of keratin fibres, and in particular of human keratin fibres such as the hair, **characterized in that** it comprises, in a medium which is suitable for dyeing, at least one compound of formula (I) as defined in any one of Claims 1 to 3, as an oxidation base.

6. Composition according to Claim 5, **characterized in that** the compound(s) of formula (I) represent(s) from 0.0005 to 12% by weight relative to the total weight of the dye composition.

7. Composition according to Claim 6, **characterized in that** the compound(s) of formula (I) represent(s) from 0.005 to 6% by weight relative to the total weight of the dye composition.

8. Composition according to any one of Claims 5 to 7, **characterized in that** the medium which is suitable for dyeing (or the support) consists of water or a mixture of water and at least one organic solvent chosen from C₁-C₄ lower alkanols, glycerol, glycols and glycol ethers, aromatic alcohols, similar products and mixtures thereof.

9. Composition according to any one of Claims 5 to 8, **characterized in that** it has a pH of between 3 and 12.

10. Composition according to any one of Claims 5 to 9, **characterized in that** it contains at least one additional oxidation base chosen from para-phenylenediamines, bis(phenyl)alkylenediamines other than the compounds of formula (I), para-aminophenols, ortho-aminophenols and heterocyclic bases.

11. Composition according to Claim 10, **characterized in that** the additional oxidation base(s) represent(s) from 0.0005 to 12% by weight relative to the total weight of the dye composition.

12. Composition according to any one of Claims 5 to 11, **characterized in that** it contains at least one coupler and/or at least one direct dye.

13. Composition according to Claim 12, **characterized in that** the coupler(s) is (are) chosen from meta-phenylenediamines, meta-aminophenols, meta-diphenols and heterocyclic couplers, and the addition salts thereof with an acid.

14. Composition according to Claim 12 or 13, **characterized in that** the coupler(s) represent(s) from 0.0001 to 10% by weight relative to the total weight of the dye composition.

15. Composition according to any one of Claims 5 to 14, **characterized in that** the addition salts with an acid are chosen from the hydrochlorides, hydrobromides, sulphates, citrates, succinates, tartrates, lactates and acetates.

16. Process for dyeing keratin fibres, and in particular human keratin fibres such as the hair, **characterized in that** at least one dye composition as defined in any one of Claims 5 to 15 is applied to these fibres and the colour is developed at acidic, neutral or alkaline pH using an oxidizing agent which is added to the dye composition just at the time of use, or which is present in an oxidizing composition which is applied simultaneously or sequentially in a separate manner.

17. Process according to Claim 16, **characterized in that** the oxidizing agent is chosen from hydrogen peroxide, urea peroxide, alkali metal bromates and persalts such as perborates and persulphates.

18. Multi-compartment dyeing device or multicompartment dyeing "kit", a first compartment of which contains a dye composition as defined in any one of Claims 5 to 15, and a second compartment of which contains an oxidizing composition.
